# FASCICULE DE BREVET EUROPEEN

(11) **EP 0 514 262 B1**
(45) Date de publication et mention de la délivrance du brevet: **28.08.1996**
(21) Numéro de dépôt: 92401303.0
(22) Date de dépôt: 13.05.1992
(51) Int. Cl.: A61F 2/16

(54) **Dispositif intra-oculaire implantable dans le sac capsulaire**
Intraokulare Vorrichtung für die Implantation in dem Kapelsack
Intra-ocular device for implanting in the capsular bag

(30) Priorité: 17.05.1991 FR 9106050
(43) Date de publication de la demande: 19.11.1992
(73) Titulaire: CORNEAL, F-75012 Paris (FR)
(72) Inventeur: Hachet, Etienne, F-54000 Nancy (FR)
(74) Mandataire: Dronne, Guy

(56) Documents cités:
- EP-A- 0 106 488
- WO-A-90/07914
- US-A- 4 494 254
- US-A- 4 710 195
- US-A- 4 804 361

## Description

La présente invention a pour objet un dispositif intra-oculaire implantable dans le sac capsulaire de l'oeil.

Les dispositifs intra-oculaires pour implantation, plus couramment appelés implants intra-oculaires, connaissent un développement important. Ils constituent des systèmes correcteurs de la vision humaine qui peuvent, dans un certain nombre de cas, se substituer à des lentilles cornéennes ou à des verres correcteurs externes. Dans d'autres cas, ces implants peuvent être combinés par exemple avec des verres correcteurs pour obtenir une correction convenable de la vision. Un implant intra-oculaire se compose essentiellement d'une partie optique de forme générale circulaire ou légèrement ovalisée qui forme le système optique correcteur proprement dit. Un implant comporte également une partie haptique qui sert à la mise en place et à la fixation de la partie optique à l'intérieur de l'oeil.

Dans les dispositifs intra-oculaires les plus récents, cette partie haptique se compose le plus souvent de deux anses flexibles partant de la périphérie de la partie optique et dont les extrémités viennent au contact de la paroi interne de l'oeil pour assurer la mise en place de la partie optique et son centrage correct par rapport à l'axe oculaire de l'oeil humain. Les anses de la partie haptique doivent présenter une certaine élasticité pour pouvoir assurer une force de maintien suffisante pour assurer la mise en place du système optique. Le plus souvent également la partie optique et la partie haptique ne forment qu'une seule et même pièce usinée dans un matériau bio-compatible.

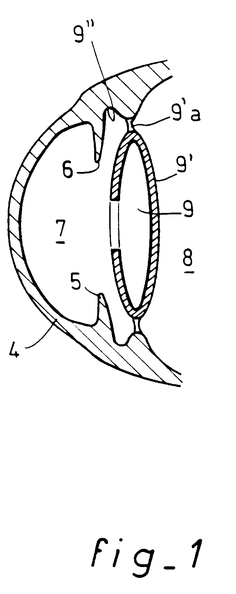

On peut distinguer trois types principaux d'implants intra-oculaires, selon la partie interne de l'oeil dans laquelle l'implant est mis en place. En se référant à la figure 1 qui est une vue en coupe verticale d'un oeil, on comprendra mieux les différents types d'implantation. Sur cette figure, on a représenté la cornée 4, ainsi que l'iris 5 qui définit ainsi la pupille 6. L'espace compris entre l'iris 5 et la partie avant de la cornée 4 limite la chambre antérieure 7. En arrière de l'iris 5, on trouve la chambre postérieure 8. Dans celle-ci, on trouve le cristallin 9 qui est enfermé dans le sac capsulaire 9′ dont la périphérie 9′a est solidaire de la cornée 4. Lorsqu'on est amené à pratiquer une opération de la cataracte, on procède à l'enlèvement du cristallin 9 qui peut se faire en laissant en place le sac capsulaire 9.

On comprend qu'ainsi la mise en place d'un implant peut se faire dans trois régions distinctes qui sont la chambre antérieure 7, la chambre postérieure 8 et le sac capsulaire 9′.

La présente invention concerne un implant intra-oculaire susceptible d'être mis en place dans le sac capsulaire 9′. Un des avantages de l'implantation dans le sac capsulaire 9′ consiste dans le fait que la partie aptique de l'implant est au contact du sac restant 9′ et non de la paroi interne de la cornée, ce qui évite ainsi les risques de lésion de la paroi interne de celle-ci. En outre, le diamètre interne du sac capsulaire est de l'ordre de 9 à 10 mm. En revanche, l'implantation dans le sac capsulaire est plus délicate, d'une part, parce que le diamètre interne du sac est relativement incertain, en général compris entre 9 et 10 mm, et que, d'autre part, le sac 9′ est relativement fragile. En conséquence, lors de la mise en place de l'implant dans le sac, il existe un risque que celui-ci ne soit déchiré et que la partie aptique de l'implant ne vienne au contact de la paroi interne de la cornée et plus précisément du sillon 9˝, ce qui entraîne un mauvais centrage et une mauvaise fixation de l'implant, en raison de la différence de diamètre entre le sac capsulaire 9′ et le sillon 9˝.

Une autre évolution importante actuelle de la chirurgie mise en oeuvre pour l'implantation intra-oculaire consiste dans le fait que l'on tend de plus en plus à pratiquer dans la cornée une incision de longueur aussi réduite que possible pour éviter l'apparition de phénomènes d'astigmatisme résultant de cette incision. Plus précisément, dans les techniques antérieures, on pratiquait une incision sur un angle d'environ 180°, ce qui permettait une introduction relativement aisée d'implants de diamètre et de dimension importantes mais entraînait trop fréquemment l'apparition d'astigmatisme. Selon les techniques plus récentes, on pratique une incision dans la cornée de l'ordre de 60°, ce qui correspond à une ouverture de 6 à 7 mm de largeur. On comprend qu'avec une telle technique il est nécessaire de disposer d'implants présentant des dimensions réduites par rapport à celles des implants utilisés précédemment.

On comprend que la technique d'implantation dans le sac capsulaire convient bien à ces techniques chirurgicales à incision réduite puisque le sac capsulaire a des dimensions réduites par rapport à la chambre antérieure ou à la chambre postérieure.

La mise en place de l'implant dans le sac capsulaire nécessite que les anses de la partie haptique présentent une élasticité importante pour éviter de déchirer le sac avec comme conséquence les inconvénients mentionnés précédemment. En outre, comme l'incision pratiquée pour l'introduction de l'implant à des dimensions réduites, il est nécessaire lors de cette introduction de replier de façon importante les anses pour que la dimension globale externe de l'implant soit inférieure aux dimensions de l'incision. Cette courbure des anses est obtenue en tenant l'implant et, plus précisément, ses anses à l'aide d'instruments chirurgicaux qui exercent localement sur ces anses une pression importante susceptible, sans précaution particulière, d'entraîner la rupture de l'extrémité des anses et donc de rendre inutilisable l'implant ainsi altéré.

Une autre difficulté consiste dans le fait que, avec des anses souples d'une longueur importante du type couramment utilisé, il est difficile de contrôler la déformation de ces anses après la mise en place de l'implant dans l'oeil. Il en résulte qu'il est difficile de contrôler le centrage correct de la partie optique du fait des possibilités de déformation dissymétrique des deux anses dans le cas où celles-ci ont à la fois une grande élasticité et une longueur relativement importante.

Les observations et essais déjà effectués ont montré que la transposition directe de la forme des anses couramment utilisées dans le cas d'implants pour chambre antérieure ou pour chambre postérieure ne permet pas d'obtenir des résultats satisfaisants avec des implants pour sac capsulaire. En effet, l'élasticité obtenue est le plus souvent insuffisante et le contact obtenu entre l'extrémité des anses et la périphérie du sac capsulaire n'est pas satisfaisant pour obtenir un centrage convenable de la partie optique dans le sac.

Le document EP-A-106.488 décrit un implant intraoculaire pour chambre postérieure qui comprend deux boucles haptiques. Chaque boucle comprend une portion radiale rigide et une portion flexible de grande longueur de telle manière que l'extrémité terminale d'une boucle soit en regard de la première extrémité de l'autre boucle. Une telle solution ne permet pas un maintien satisfaisant de l'implant dans l'oeil.

Un objet de l'invention est de fournir un dispositif intra-oculaire implantable dans le sac capsulaire qui permet, d'une part, une implantation à l'aide d'une incision dans la cornée de longueur réduite et qui, d'autre part, permet un maintien et un centrage de la partie optique corrects dans le sac capsulaire.

Pour atteindre ce but, le dispositif intra-oculaire pour implantation dans le sac capsulaire, selon l'invention, comprend une partie optique de forme sensiblement circulaire et une partie haptique constituée par deux anses flexibles incurvées, chacune présentant une première extrémité libre destinée à venir en contact avec la paroi du sac capsulaire et une deuxième extrémité raccordée à la périphérie de la partie optique, chaque anse comportant une première portion sensiblement radiale et relativement rigide présentant une extrémité se terminant sur un deuxième cercle concentrique à la partie optique et une deuxième portion partant de ladite extrémité de ladite première portion sensiblement tangentiellement audit deuxième cercle et il se caractérise en ce que la partie optique a un diamètre compris entre 4,5 et 6 mm, en ce que l'ensemble des deux anses au repos, est situé à l'intérieur d'un premier cercle concentrique à la partie optique et présentant un diamètre compris entre 11,5 et 13 mm, le diamètre dudit deuxième cercle étant égal à 7 mm , ladite deuxième portion présentant une courbure sensiblement constante et comportant une partie courante dont la largeur e est sensiblement constante et une extrémité terminale dont la largeur e' est comprise entre 1,4e et 1,8e.

Par sensiblement tangente, il faut entendre que l'anse fait avec la tangente au cercle un angle inférieur à 10°.

On obtient ainsi deux anses qui sont suffisamment flexibles pour pouvoir être repliées et introduites dans l'oeil à l'aide d'une incision de longueur réduite tout en présentant une courbure et une flexion suffisante pour s'adapter aux différents diamètres du sac capsulaire tout en assurant une bonne fixation et un bon centrage de la partie optique.

Avec la caractéristique citée en dernier lieu dans la revendication 1, on obtient des anses dont l'extrémité est renforcée permettant ainsi l'utilisation des instruments chirurgicaux avec une grande sécurité et sans risque de rupture tout en autorisant une courbure et une flexibilité suffisantes en localisant la courbure dans la partie courante de l'anse, ou plus précisément de la deuxième portion de l'anse.

D'autres caractéristiques et avantages de l'invention apparaîtront plus clairement à la lecture de la description qui suit d'un mode préféré de réalisation de l'invention, donné à titre d'exemple non limitatif. La description se réfère aux figures annexées sur lesquelles :
- la figure 1, déjà décrite, montre en coupe longitudinale un oeil humain afin de montrer les différentes possibilités d'implantation ;
- la figure 2 est une vue de face d'un dispositif intra-oculaire selon l'invention ; et
- la figure 3 est une vue de côté de l'implant de la figure 2.

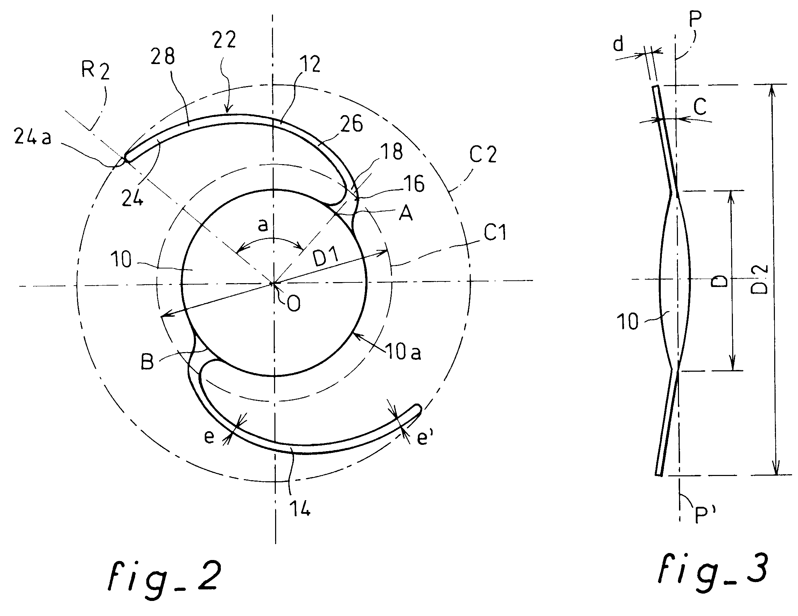

En se référant aux figures 2 et 3, on va décrire un mode préféré de réalisation du dispositif intra-oculaire pour implantation dans le sac capsulaire, que l'on appellera ultérieurement par simplification implant. L'implant comprend une partie optique constituée par une lentille convergente 10 de centre 0 et de diamètre D. Dans l'exemple particulier considéré, le diamètre est égal à 5,5 mm mais de façon plus générale ce diamètre peut être compris entre 4,5 et 6 mm. La partie haptique de l'implant est constituée par deux anses respectivement référencées 12 et 14, chaque anse part de la périphérie 10a de la lentille 10 en des points A et B qui sont diamétralement opposés. Les deux anses 12 et 14 sont sensiblement identiques et on se contentera donc de décrire en détail l'anse 12. Celle-ci se compose tout d'abord d'une première portion 16 disposée selon un prolongement d'un rayon de la lentille 10. Cette première portion radiale présente une largeur non négligeable conférant à cette portion une certaine rigidité. L'extrémité 18 de la portion radiale 16 est disposée sur un cercle C1 concentrique à la lentille 10 et dont le diamètre D1 est sensiblement égal à 7 mm. On rappelle que le diamètre d'une lentille pour un implant classique pour chambre antérieure ou chambre postérieure est sensiblement égal précisément à 7 mm. L'anse 12 est constituée également par une deuxième portion 22 dont une première extrémité est raccordée à la portion radiale 16 et dont l'extrémité terminale 24 est destinée à venir en contact avec la paroi interne du sac capsulaire afin d'assurer à la fois la fixation de la partie optique 10 et son centrage correct. La deuxième portion de l'anse est constituée, d'une part, par une partie courante 26 directement raccordée à la portion radiale 16 et, d'autre part, une portion terminale 28. L'ensemble de la deuxième portion 22 de l'anse 12 présente une courbure sensiblement constante. De plus, on voit que la portion 22 part sensiblement tangentiellement au cercle de diamètre D1 en faisant avec celui-ci un angle réduit inférieur à 10°. On voit également que la largeur e de la portion courante 26 de l'anse, c'est-à-dire sa dimension dans le plan de la lentille 10 est sensiblement inférieure à la largeur e′ de la portion terminale d'extrémité 28. Conformément à la revendication 1, e′ est compris entre 1,4 fois e et 1,8 fois e. Dans l'exemple considéré, e est égal à 0,14 mm et e′ est égal à 0,20 mm.

En outre, on voit que l'ensemble des anses 12 et 14 est inscrit à l'intérieur d'un cercle C2 concentrique à la lentille 10 dont le diamètre D2 est égal, dans l'exemple considéré, à 12 mm. Plus généralement, le diamètre D2 est compris entre 11,5 et 13 mm. Bien sûr, sur la figure 2, on a représenté les anses 12 et 14 au repos, c'est-à-dire dans leur position où elles ne sont soumises à aucun effort de flexion. On note également que l'extrémité terminale 24 de chacune des anses se termine avec une légère angulation par rapport au cercle C2. Plus précisément, cet angle est déterminé pour que, lorsque l'implant est mis en place dans le sac 9', la portion terminale de chaque anse fléchie soit sensiblement tangente à la périphérie de la paroi interne du sac, c'est-à-dire à un cercle de diamètre compris entre 9 et 10 mm.

La figure 2 montre également que l'angle au centre a limité par la portion radiale 16 et par le rayon R2 joignant le centre 0 à l'extrémité 24a du l'anse 12 est, dans le cas particulier considéré, égal à 93°, Plus généralement, l'angle a est compris entre 85 et 100°.

Si l'on se réfère maintenant à la figure 3, on voit que l'épaisseur d de chaque anse 12 ou 14 est sensiblement constante sur toute la longueur des anses et vaut dans le cas particulier 0,15 mm. En outre, on voit que la direction générale de chaque anse fait un angle c avec le plan PP' de la lentille 10. Cet angle c est dans l'exemple considéré égal à 10 degrés.

Il découle de la description précédente que l'implant selon l'invention s'adapte parfaitement à l'implantation dans le sac capsulaire selon la technique de l'incision réduite. D'une part, du fait de la présence des premières portions radiales 16, les deuxièmes portions de chaque anse se présentent sensiblement comme dans le cas des anses pour un implant de type classique, c'est-à-dire de diamètre de partie optique de l'ordre de 7 mm. Bien entendu, la longueur de chaque deuxième portion d'anse a été limitée pour s'adapter au diamètre externe D2 de la partie haptique. En outre, du fait que les parties terminales des anses ont une largeur e' sensiblement supérieure à celle de la portion courante, on obtient dans les portions terminales une résistance mécanique supérieure tout en autorisant grâce à la largeur réduite de la portion courante une flexion satisfaisante des anses. Du fait de cette portion de largeur accrue, il est possible de saisir efficacement l'implant à l'aide d'instruments chirurgicaux sans risquer d'entraîner la rupture des anses. De plus cette portion de largeur plus importante diminue sensiblement les risques de percer le sac capsulaire.

En outre, la longueur totale de chaque anse est relativement réduite par rapport aux implants de type connu à anse souple grâce à la valeur relativement limitée de l'angle au centre a. Cette longueur relativement réduite de chaque anse permet de mieux contrôler la déformation élastique de ces anses pour se conformer au diamètre interne du sac capsulaire tout en maintenant un bon centrage de l'implant. Enfin, il faut remarquer que, grâce à la présence des portions radiales 16 des anses et à la forme particulière de celle-ci, les deuxièmes portions des anses ne présentent qu'une angulation relativement faible par rapport au cercles C1 ou C2. On obtient ainsi une très bonne flexibilité de la deuxième portion des anses qui se plient essentiellement dans leur partie courante, entre l'extrémité plus large et la portion radiale relativement rigide.

## Revendications

1. Dispositif intraoculaire pour implantation dans le sac capsulaire, comprenant une partie optique (10) de forme sensiblement circulaire et une partie haptique constituée par deux anses flexibles incurvées (12, 14), chacune présentant une première extrémité libre destinée à venir en contact avec la paroi du sac capsulaire et une deuxième extrémité (18) raccordée à la périphérie de la partie optique, chaque anse (12, 14) comportant une première portion sensiblement radiale (16) et relativement rigide présentant une extrémité se terminant sur un deuxième à la cercle (C1) concentrique à la partie optique et une deuxième portion (22) partant de ladite extrémité de ladite première portion sensiblement tangentiellement audit deuxième cercle (C1), caractérisé en ce que la partie optique (10) a un diamètre (D) compris entre 4,5 et 6 mm, en ce que l'ensemble des deux anses (12, 14) au repos est situé à l'intérieur d'un premier cercle (C2) concentrique à la partie optique et présentant un diamètre compris entre 11,5 et 13 mm, le diametre dudit deuxième cercle (C1) étant égal à 7 mm, ladite deuxième portion présentant une courbure sensiblement constante et comportant une partie courante (26) dont la largeur e est sensiblement constante et une extrémité terminale (28) dont la largeur e' est comprise entre 1,4e et 1,8e.

2. Dispositif intraoculaire selon la revendication 1, caractérisé en ce que ladite portion terminale (28) fait avec ledit premier cercle C2 un angle tel que, lorsque ledit dispositif est mis en place dans le sac capsulaire, ladite portion terminale fléchie soit sensiblement tangente à un cercle de diamètre compris entre 9 et 10 mm correspondant aux dimensions internes du sac capsulaire.

3. Dispositif intraoculaire selon l'une quelconque des revendications 1 et 2, caractérisé en ce que lesdites anses (12, 14) ont une épaisseur sensiblement constante.

4. Dispositif intraoculaire selon l'une quelconque des revendications 1 à 3, caractérisé en ce que l'angle au centre (a) limité par ladite première portion radiale (16) et par le rayon (R2) passant par l'extrémité (24a) de ladite deuxième portion est compris entre 85 et 100°.

5. Dispositif intraoculaire selon l'une quelconque des revendications 1 à 4, caractérisé en ce que ladite première portion radiale (16) de chaque anse (12, 14) a une largeur sensiblement supérieure à celle de la portion terminale (28).

6. Dispositif intraoculaire selon l'une quelconque des revendications 1 à 5, caractérisé en ce que chaque anse (12, 14) fait avec le plan (PP') de la partie optique (10) un angle de l'ordre de 10 degrés.

## Claims

1. An intraocular device for implantation in the capsular sac, comprising an optical part (10) substantially circular in shape and a haptic part constituted by two flexible curved arms (12, 14) each having a first free extremity intended to come into contact with the wall of the capsular sac and a second extremity (18) connected to the periphery of the optical part, each arm (12, 14) comprising a first substantially radial and relatively rigid portion (16) having one extremity that ends at a second circle (C1) concentric to the optical part and a second portion (22) starting from said extremity of said first portion, substantially tangentially to said second circle (C1), characterised in that the optical part (10) has a diameter (D) of between 4.5 and 6 mm, in that the two arms (12, 14) together when at rest are located within a first circle (C2) concentric to the optical part and having a diameter of between 11.5 and 13 mm, the diameter of said second circle (C1) being equal to 7 m, said second portion having a substantially constant curvature and comprising an extended part (26), the width e of which is substantially constant and a terminal extremity (28), the width e' of which is between 1.4e and 1.8e.

2. An intraocular device according to Claim 1, characterised in that said terminal portion (28) forms an angle with said first circle (C2) such that, when said device is positioned in the capsular sac, said terminal portion, being flexed, is substantially tangential to a circle with a diameter of between 9 and 10 mm corresponding to the internal dimensions of the capsular sac.

3. An intraocular device according to any one of Claims 1 and 2, characterised in that said arms (12, 14) have a substantially constant thickness.

4. An intraocular device according to any one of Claims 1 to 3, characterised in that the angle at the centre (a) limited by said first radial portion (16) and by the radius (R2) passing through the extremity (24a) of said second portion is comprised between 85 and 100°,

5. An intraocular device according to any one of Claims 1 to 4, characterised in that said first radial portion (16) of each arm (12, 14) has a width substantially greater than that of the terminal portion (28).

6. An intraocular device according to any one of Claims 1 to 5, characterised in that each arm (12, 14) forms an angle with the plane (PP') of the optical part (10), of the order of 10 degrees.

## Patentansprüche

1. Intraokulare Vorrichtung für die Implantation im Kapselsack, enthaltend:
einen optischen Teil (10) mit im wesentlichen kreisförmiger Form und einen Halteteil, der aus zwei flexiblen gekrümmten Bügeln (12, 14) gebildet ist, von denen jeder ein erstes freies Ende aufweist, das in Kontakt mit der Wand des Kapselsacks gelangt, und ein zweites Ende (18), das mit dem Rand des Halteabschnitts verbunden ist, wobei jeder Bügel (12, 14) einen im wesentlichen radial verlaufenden und relativ starren Abschnitt (16) aufweist, von dem ein Ende in einen konzentrisch mit dem optischen Teil verlaufenden zweiten Kreis (C1) mündet, und einen zweiten Abschnitt (22), der von dem Ende des ersten Abschnitts im wesentlichen tangential zu dem zweiten Kreis (C1) ausgeht,
dadurch **gekennzeichnet,** daß
der optische Teil (10) mit einem Durchmesser (D) von 4,5 bis 6 mm ausgebildet ist,
daß die beiden Bügel (12, 14) in Ruhestellung innerhalb eines ersten Kreises (C2) angeordnet sind, der konzentrisch zu dem optischen Teil verläuft und einen Durchmesser zwischen 11,5 und 13 mm aufweist,
der Durchmesser des zweiten Kreises (C1) gleich 7 mm ist,
der zweite Abschnitt eine im wesentlichen konstante Krümmung aufweist und einen Biegeabschnitt (26) aufweist, dessen Breite e im wesentichen konstant ist, und einen Endabschnitt (28), dessen Breite e' zwischen 1,4 x e und 1,8 x e liegt.

2. Intraokulare Vorrichtung nach Abspruch 1, dadurch gekennzeichnet, daß der Endabschnitt (28) mit dem ersten Kreis (C2) einen Winkel einschließt, derart, daß dann, wenn die Vorrichtung in dem Kapselsack angeordnet ist, der gebogene Endabschnitt im wesentlichen tangential zu einem Kreis mit einem Durchmesser zwischen 9 und 10 mm gemäß den Innenabmessungen des Kapselsacks verläuft.

3. Intraokulare Vorrichtung nach Abspruch 1 oder 2, dadurch gekennzeichnet, daß die Bügel (12, 14) eine im wesentlichen konstante Dicke aufweisen.

4. Intraokulare Vorrichtung nach einem der Absprüche 1 bis 3, dadurch gekennzeichnet, daß der durch den ersten Radialabschnitt (16) und der durch den durch das Ende (24a) des zweiten Abschnitts verlaufenden Strahl (R2) begrenzte Mittenwinkel (a) in einem Bereich zwischen 85° und 100° liegt.

5. Intraokularvorrichtung nach einem der Ansprüche 1 bis 4, dadurch gekennzeichnet, daß der erste Radialabschnitt (16) jedes Bügels (12, 14) eine im wesentlichen größere Breite als der Endabschnitt (28) aufweist.

6. Intraokulare Vorrichtung nach einem der Ansprüche 1 bis 5, dadurch gekennzeichnet, daß jeder Bügel (12, 14) mit der Ebene (PP') des optischen Teils (10) einen Winkel in der Größenordnung von 10° einschließt.
